# EUROPEAN PATENT SPECIFICATION

(11) **EP 3 407 879 B1**
(45) Date of publication and mention of the grant of the patent: **06.01.2021**
(21) Application number: 17703933.6
(22) Date of filing: 25.01.2017
(51) Int. Cl.: A61K 31/195, A61K 31/728, A61P 27/02, A61K 9/00

(54) **OPHTHALMIC GABAPENTIN FOR THE TREATMENT OF CORNEAL ULCERS**
OPHTHALMISCHES GABAPENTIN ZUR BEHANDLUNG VON HORNHAUTGESCHWÜREN
GABAPENTINE OPHTALMIQUE POUR LE TRAITEMENT D'ULCÈRES CORNÉENS

(30) Priority: 29.01.2016 IT UB20160121
(43) Date of publication of application: 05.12.2018
(60) Divisional application: 20160507.8
(73) Proprietor: Sooft Italia S.p.A., 63833 Montegiorgio (FM) (IT)
(72) Inventor: BIONDI, Piero, I-63833 Montegiorgio (FM) (IT); STAGNI, Edoardo, I-63833 Montegiorgio (FM) (IT)
(74) Representative: Bianchetti Bracco Minoja S.r.l.
(86) International application number: PCT/EP2017/051463
(87) International publication number: WO 2017/129577

(56) References cited:
- EP-A1- 2 316 420
- MARC SCHARGUS ET AL: "Treatment of Severe Ocular Surface Disorders with Albumin Eye Drops", JOURNAL OF OCULAR PHARMACOLOGY AND THERAPEUTICS., vol. 31, no. 5, 8 April 2015 (2015-04-08), pages 291-295, XP055306794, US ISSN: 1080-7683, DOI: 10.1089/jop.2014.0161
- DATABASE EMBASE [Online] ELSEVIER SCIENCE PUBLISHERS, AMSTERDAM, NL; December 2013 (2013-12), FAN J -H ET AL: "Effect of sodium hyaluronate in treating fungal corneal ulcer", XP002762449, Database accession no. EMB-2014066172
- GANDOLFI S A ET AL: "Low-molecular-weight sodium hyaluronate in the treatment of bacterial corneal ulcers", GRAEFE'S ARCHIVE FOR CLINICAL AND EXPERIMENTAL OPHTHALMOLOGY, SPRINGER VERLAG, DE, vol. 230, no. 1, 1992, pages 20-23, XP009095907, ISSN: 0721-832X, DOI: 10.1007/BF00166757
- SONAL S TULI ET AL: "Science and Strategy for Preventing and Managing Corneal Ulceration Clinical Science", OCULAR SURFACE, vol. 5, no. 1, January 2007 (2007-01), pages 23-39, XP055306811, ISSN: 1542-0124

## Description

The present invention relates to ophthalmic formulations of gabapentin for use in the treatment of corneal ulcers.

### Prior art

The cornea, a structure at the front of the eye which comes into direct contact with the outside environment, is a multilayered tissue consisting of epithelial cells on the surface, below which are Bowman membrane, the corneal stroma, Descemet membrane and the endothelial cell layer.

The cornea is a normally avascular tissue that receives nutrients from the tear film, the capillaries of the limbus and the aqueous humor present in the anterior chamber of the eye; it has no immunocytes or lymphatic tissue, and is therefore transparent.

However, in order to receive the correct intake of nutrients and oxygen and perform its multiple functions as effectively as possible, the cornea is the most innervated tissue in the body (Invest Ophthalmol Vis Sci. 2015 Aug; 56(9):5417-23. Imaging of the Corneal Subbasal Whorl-like Nerve Plexus: More Accurate Depiction of the Extent of Corneal Nerve Damage in Patients With Diabetes. Utsunomiya T et al.; Exp Eye Res. 2004 Mar; 78(3):513-25. Neural basis of sensation in intact and injured corneas. Belmonte C et al.).

Specifically, the corneal innervation is mainly the sensory type, consisting of long ciliary nerve endings deriving from the nasociliary branch of the ophthalmic branch of the trigeminal nerve. In the cornea, they partly form a deep plexus above the endothelium, and partly cross the corneal endothelium and project perpendicularly into the stroma to form a (subepithelial) plexus below the corneal epithelium. The latter plexus, called the sub-basal nerve plexus, consists of the small nerves that cross the limiting front membrane and project as free nerve endings between the epithelial cells.

This structure high sensitivity to pain and heat probably serves to activate the palpebral reflex, and therefore all behaviour designed to prevent harmful stimuli, so as to protect the eye from possible traumas. The corneal nerve fibers release neuropeptides, which have a neuromodulating effect on the physiology of the corneal epithelium (proliferation, differentiation, migration and cell adhesion) and are also crucial to correct physiology and epithelial healing. Absence of normal neurotrophic activity leads to the formation of ulcerative or degenerative corneal defects.

The majority of the sensory corneal fibers, called polymodal nociceptors, respond to mechanical and heat stimuli, irritant exogenous chemicals and a wide variety of endogenous chemical mediators (protons, potassium ions, ATP, prostaglandins and other metabolites of arachidonic acid, amino acids, amines, cytokines, kinins, growth factors, etc.) released by damaged corneal tissue. During inflammation, the mediators released at local level stimulate the polymodal nociceptors, leading to a continuous discharge that produces a long-lasting pain sensation, with significant discomfort for the patient.

A reduction in the pain threshold, increased reactivity and greater spontaneous activity of the nerve fibers caused by repeated harmful stimuli are part of the "sensitization" process, a specific property present in the nociceptive neurons of all the body deep and surface tissues, including the corneal tissues. The phenomenon of sensitization results from a variety of pathogenetic mechanisms that develop successively in proportion to the extent of the damage, with the result that the reactivity of the afferent corneal neurons to mechanical and chemical stimuli becomes abnormal, and they are activated either spontaneously or in response to stimuli which, under normal conditions, would not excite intact nerve endings (Ocul Surf. 2004 Oct;2(4):248-53. Nerves and sensations from the eye surface. Belmonte C et al.).

The corneal nerve fibers use various neurotransmitters, one of the most important of which is GABA (γ amino-butyric acid). GABA has trophic effects on tissues that possess GABAergic receptors, and promotes the growth of neurons and nerve fibers (J Neurophysiol. 2003 Nov;90(5):2837-49. GABA(A) receptor activation modulates corneal unit activity in rostral and caudal portions of trigeminal subnucleus caudalis. Hirata H1, Okamoto K, Bereiter DA).

The common characteristic of various corneal diseases, which may have very different etiologies, is the formation of an ulcer, often recurrent and not sensitive to the various existing treatments, caused by chronic inflammation with periodic flare-ups. Said diseases, which are often "rare", include neurotrophic keratitis and ocular pemphigoid.

Neurotrophic keratitis is a degenerative disease of the cornea that affects one person in 10,000. There is currently no treatment for the disease.

Cicatricial pemphigoid, also called mucous membrane pemphigoid (MMP), is a rare systemic disease with an autoimmune etiology, characterised by the presence of bullous, inflammatory lesions of the mucosal tissues.

The mucosae involved are mainly those of the conjunctiva (also affecting the cornea in nearly all cases), the oral cavity and the genital apparatus. The eye surface is involved in 70% of cases.

Cicatricial pemphigoid is a rare disease which mainly affects individuals between 60 and 70 years old, and women more frequently than men. Local treatment has proved ineffective in preventing the progression of conjunctival scarring. No local treatment able to halt corneo-conjunctival fibrosis exists, and once fibrosis has occurred, it does not regress. The local medicaments used are eyedrops containing cortisone, cyclosporin, antibiotics or artificial tears. Corneal transplants have been proposed as an alternative to the pharmacological approach, but have given disappointing results in patients with ocular pemphigoid. As already stated, corneal ulcer is a symptom common to all these disorders.

Corneal ulcer refers to the condition wherein the corneal stroma, mainly consisting of collagen and keratinocytes, is lysed and degraded by activation and hypersecretion of collagenolytic enzymes. Other enzymes able to promote the formation of corneal ulcers are microbial collagenases and matrix metalloproteases (MMP). When the cornea is infected by bacteria, microbial collagenase is secreted, which directly degrades the collagen of the stroma, thus causing corneal ulcers. Simultaneously, the enzymes and toxins secreted by the micro-organisms become biological signals that stimulate activation of the corneal stromal cells. The activated corneal stromal cells produce inactive MMPs (proMMPs). They then become active MMPs, contributing to the formation of corneal ulcers (control of the activity of activated MMPs depends on the concentration of TIMPs (tissue inhibitors of metalloproteinase). The variations in the extracellular environment caused by the breakdown of stromal collagen also promote infiltration of inflammatory cells, thus giving rise to a vicious circle, resulting in further activation of the keratinocytes and increased degradation of the corneal stroma. When the bacteria are killed by antibiotics, the secretion of microbial collagenase is suppressed, and degradation of the corneal stroma terminates. However, despite the suppression of the stimulus starting from the microbial collagenases, clinical progression of the corneal ulcer is usually observed due to continued activation of the corneal stromal cells resulting from the continued inflammatory stimulus, self-generated by the sensitization of the nociceptive corneal nerve fibers that continue to conduct pain information even when the pathogenic noxa, whatever it may be, is no longer present.

The only substances currently able to interrupt said vicious circle are steroids (Nishida Teruo et al. ed., "Monthly ophthalmology therapeutic practice (vol. 79) pharmacotherapeutics of corneal and conjunctival diseases", 1st printing, Bunkodo, January 2002, pp. 9-11) which, however, also have serious side effects. Their use in the treatment of neurotrophic keratitis and corneal ulcers often gives rise to the opposite effect, and aggravates the condition.

### Description of the invention

The Applicant has carried out intensive studies in an attempt to formulate a product for topical use that disrupts said vicious circle (pathogenic noxa - inflammation - production of MMPs and other lytic enzymes - pain - inflammation - further production of MMPs and other lytic enzymes - aggravation of corneal ulcer) and thus effectively treats corneal ulcers without recourse to steroids.

It has been found that topical administration of gabapentin or pharmaceutically compatible salts thereof, a medicament which has been used for some time to treat neuropathic pain, effectively solves the problem of treating corneal ulcers of various origins, especially those resulting from pemphigoid and neurotrophic keratopathy.

Instillation of sterile solutions of gabapentin with concentrations ranging from 0.1 to 5% by weight, preferably 0.5% by weight, into the eyes of patients suffering from corneal ulcers repairs the corneal lesions rapidly and without significant side effects. Particularly suitable forms of administration are eyedrops containing, in addition to the active ingredient, amino acids (in particular glycine, leucine, proline and lysine hydrochloride) and viscosity-controlling agents, preferably hyaluronic acid, hypromellose and cellulose derivatives, or xyloglucans such as tamarind seed polysaccharide (TSP).

Hyaluronic acid of extractive origin or obtained by fermentation, in percentages ranging from 0.1 to 0.5% by weight, is preferred. Formulations of gabapentin or salts thereof suitable for ophthalmic administration were described in EP 2851063 and EP 2316420 which, however, relate to different therapeutic indications, unrelated to the treatment of corneal ulcers which forms the subject of the present invention.

The treatment according to the invention involves instilling 1-2 eyedrops into the eye one to ten times a day, depending on the severity of the disease and the type of lesion. Treatment with gabapentin can be combined with the administration of other medicaments such as antibiotics, antivirals, antibacterials, cortisone, non-steroidal anti-inflammatory drugs, antihistamines or artificial tears.

Eyedrops containing gabapentin or other equivalent topical formulations can be used as an emergency treatment to provide pain relief as well as beneficial effects on the corneal repair mechanism, or as a maintenance treatment.

Gabapentin exerts re-epithelialising activity at concentrations ranging from 5 µg/ml to 0.1 mg/ml, as demonstrated by experiments conducted *in vitro* on immortalized rabbit corneal cells (SIRC) and immortalized retinal pigmented cells (ARPE 19).

Said cells were used for wound-healing experiments wherein gabapentin proved effective at the concentration of 0.01 mg/ml.

The *in vitro* activity on cell cultures was confirmed in tests conducted *in vivo* on rabbit eyes subjected to corneal de-epithelialization. The protocols and results of said experiments are reported in the examples.

Gabapentin, at the concentration of 10 µg/ml, also proved to inhibit the production of pro-inflammatory cytokines TNF-α, IL1-β, b-actin, IL-6 and IL-10 in SIRC cell cultures subjected to lipopolysaccharide stimulus (LPS, 1 µg)/ml). At the same concentration, gabapentin instilled topically into rabbits treated with LPS in experimental uveitis models inhibits the production of TNF-α, iNOS and PGE2 in the cornea, conjunctiva, ciliary bodies, aqueous humor and tears.

Preliminary clinical tests on patients suffering from corneal ulcers have demonstrated the efficacy of ocular administration of gabapentin. The results of some cases are summarized in the Table below.

| Patient | Diagnosis | Treatment | Results |
|---|---|---|---|
| Woman, 77 years | mucocicatricial pemphigoid at 3rd bilateral stage with right eye aggravated by persistent epithelial defect (PED) complicated by corneal neovascularization | single-dose eyedrops containing 0.15% hyaluronic acid, amino acids and 0.5% gabapentin 10 times/day | complete repair on 24th day. Neovascularization with sub-total regression, with maintenance treatment comprising hyaluronic acid and dexamethasone eyedrops |
| Girl, 2 years | severe bilateral corneal dystrophy in lower hemicornea | single-dose eyedrops containing 0.15% hyaluronic acid, amino acids and 0.5% gabapentin, 4 times a day for 2 weeks | gradual improvement with regression of bilateral corneal ulceration |
| Boy, 4 years | mono lateral neuropathic corneal ulcer | 0.15% hyaluronic acid, amino acids and 0.5% gabapentin, 4 times a day for 4 days | Lesion repair |
| Girl, 3 years | recurrent bilateral corneal oedema complicated by corneal ulcer | 0.15% hyaluronic acid, amino acids and 0.5% gabapentin, 4 times a day for 7 days | Lesion repair |
| Woman, 85 years | dry eye, with almost total de-epithelialization and major corneal oedema in stroma | 0.15% hyaluronic acid, amino acids and 0.5% gabapentin, 5 times a day for 6 days | Lesion repair |

Some formulation examples are set out below, together with the results of the wound-healing activity *in vitro* and *in vivo.*

### Example 1

| **per 100g of product** | **% w/w** |
|---|---|
| Gabapentin | 0.500 g |
| Hyaluronic acid sodium salt | 0.150 g |
| Sodium chloride | 0.190 g |
| Buffered solution | q.s. to 100 g |

### Example 2

| **per 100 g of product** | **% w/w** |
|---|---|
| Gabapentin | 0.500 g |
| Hyaluronic acid sodium salt | 0.150 g |
| Sodium chloride | 0.190 g |
| L-proline | 0.0752 g |
| L-glycine | 0.100 g |
| L-lysine hydrochloride | 0.014 g |
| L-leucine | 0.0108 g |
| Buffered solution | q.s. to 100 g |

### Example 3

| **per 100 g of product** | **% w/w** |
|---|---|
| Gabapentin | 0.500 g |
| Hyaluronic acid sodium salt | 0.150 g |
| Sodium chloride | 0.3267 g |
| L-proline | 0.0752 g |
| L-glycine | 0.100 g |
| L-lysine hydrochloride | 0.014 g |
| L-leucine | 0.0108 g |
| Riboflavin sodium phosphate | 0.5000 g |
| Buffered solution | q.s. to 100 g |

### Example 4 - Wound healing in vitro

The SIRC and ARPE 19 cells were seeded in a 24-well multiwell plate, in wells coated with fibronectin and type I collagen. When confluence was reached, the cells were serum-starved. The cell monolayer was scratched with a p200 tip, and after suitable washes in PBS1X the cells were treated with gabapentin at concentrations of 0.01 mg/ml and 0.05 mg/ml for 24, 48 and 72 h. The scratch repair process was then monitored photographically.

The results set out in Tables 1 and 2 prove that gabapentin exerts a re-epithelialising effect over time (24 h - 48 h - 72 h).

**Table 1**

| *% Wound Closure (mean*±*S.D.)* | **CTRL** | GBP 0.01 mg/ml **(0.001%)** | GBP 0.0 mg/ml **(0.005%)** |
|---|---|---|---|
| TO h | 0 | 0 | 0 |
| 24 h | 15±0.5 | 24±3.5* | 21±5.5 |
| 48 h | 34±7 | 5 0±7 * | 41±1 |
| 72 h | 57±5 | 70±5* | 61±3 |

**Table 2**

| *% Wound Closure (mean±S.D.)* | **CTRL** | GBP 0.01 mg/ml **(0.001%)** | GBP 0.02 mg/ml **(0.002%)** |
|---|---|---|---|
| **T0 h** | 0 | 0 | 0 |
| **24 h** | 12±1 | 14±3 | 19±3 |
| **48 h** | 27±4 | 35±3 | 35±4 |

### Example 5 - Wound healing in vivo

To evaluate the re-epithelialising effect of gabapentin, 10 rabbit eyes were subjected to corneal de-epithelialization with an Algerbrush II instrument; in particular, the rabbits were divided into three groups:
- 2 eyes instilled with sterile PBS (carrier)
- 4 eyes instilled with 1 mg/ml gabapentin (0.1%);
- 4 eyes instilled with 5 mg/ml gabapentin (0.5%).

The protocol required 4 daily instillations (of 50 µl each) at 2-hour intervals. The damaged corneas were examined daily (T0, T24, T48 and T72) after instilling 1% fluorescein. The rabbits were euthanized 72 hours after the lesion, and the tissues (cornea, tears and aqueous humor) were removed.

### Results

The fluorescein highlighted the damaged area (corneal epi-off). The areas were measured with IMAGE J software. The results demonstrated that 0.1% gabapentin (GBP) has a re-epithelialising effect 48 hours after the lesion.

**Table 3**

| *% Wound Closure (mean±S.D.)* | **CTRL** | **GBP 1 mg/ml (0.1%)** | **GBP 5 mg/ml (0.5%)** |
|---|---|---|---|
| TO h | 0 | 0 | 0 |
| 24 h | 35±1.7 | 39±13 | 38±15 |
| 48 h | 78±5 | **90±11** | 75±10 |
| 72 h | 100±4 | 100±10 | 100±12 |

## Claims

1. Ophthalmic formulations comprising gabapentin for use in the treatment of corneal ulcers.

2. Formulations for use according to claim 1, further comprising viscosity-increasing agents.

3. Formulations for use according to claim 2, wherein the viscosity-increasing agent is hyaluronic acid.

4. Formulations for use according to claims 1-3, further comprising amino acids.

5. Formulations for use according to claims 1-4, in the form of eyedrops.

6. Formulations for use according to claims 1-5, wherein gabapentin is present in concentrations ranging from 0.1 to 5% by weight.

7. Formulations for use according to claims 1-6, wherein the viscosity-increasing agent is present in concentrations ranging from 0.1 to 0.5% by weight.

8. Formulations for use according to claims 1-7, wherein the ulcers are associated with mucocicatricial pemphigoid with epithelial lesions complicated by corneal neovascularisations or with keratopathy caused by neurogenic and neurotrophic impairment.

## Patentansprüche

1. Ophthalmische Formulierungen, umfassend Gabapentin zur Verwendung bei der Behandlung von Hornhautulcera.

2. Formulierungen zur Verwendung nach Anspruch 1, des Weiteren umfassend Viskosität-erhöhende Agenzien.

3. Formulierungen zur Verwendung nach Anspruch 2, wobei das Viskosität-erhöhende Agens Hyaluronsäure ist.

4. Formulierungen zur Verwendung nach den Ansprüchen 1 bis 3, des Weiteren umfassend Aminosäuren.

5. Formulierungen zur Verwendung nach den Ansprüchen 1 bis 4, in der Form von Augentropfen.

6. Formulierungen zur Verwendung nach den Ansprüchen 1 bis 5, wobei Gabapentin in Konzentrationen im Bereich von 0,1 bis 5 Gew.-% vorliegt.

7. Formulierungen zur Verwendung nach den Ansprüchen 1 bis 6, wobei das Viskosität-erhöhende Agens in Konzentrationen im Bereich von 0,1 bis 0,5 Gew.-% vorliegt.

8. Formulierungen zur Verwendung nach den Ansprüchen 1 bis 7, wobei die Ulcera mit vernarbendem Schleimhautpemphigoid mit epithelialen Läsionen, die durch Neovaskularisationen der Hornhaut erschwert werden, oder mit Keratopathie, die durch neurogene und neurotrophe Beeinträchtigung verursacht wird, in Zusammenhang stehen.

## Revendications

1. Formulations ophtalmiques comprenant de la gabapentine pour l'utilisation dans le traitement des ulcères de la cornée.

2. Formulations pour l'utilisation selon la revendication 1, comprenant en outre des agents d'accroissement de la viscosité.

3. Formulations pour l'utilisation selon la revendication 2, l'agent d'accroissement de la viscosité étant l'acide hyaluronique.

4. Formulations pour l'utilisation selon les revendications 1 à 3, comprenant en outre des acides aminés.

5. Formulations pour l'utilisation selon les revendications 1 à 4, sous la forme de gouttes ophtalmiques.

6. Formulations pour l'utilisation selon les revendications 1 à 5, la gabapentine étant présente en des concentrations s'étendant de 0,1 à 5 % en poids.

7. Formulations pour l'utilisation selon les revendications 1 à 6, l'agent d'accroissement de la viscosité étant présent en des concentrations s'étendant de 0,1 à 0,5 % en poids.

8. Formulations pour l'utilisation selon les revendications 1 à 7, les ulcères étant associés à la pemphigoïde cicatricielle à lésions épithéliales compliquées par des néovascularisations cornéennes ou à la kératopathie causée par des troubles neurogéniques et neurotrophiques.
